# EUROPÄISCHE PATENTSCHRIFT

(11) **EP 2 196 455 B1**
(45) Veröffentlichungstag und Bekanntmachung des Hinweises auf die Patenterteilung: **26.09.2012**
(21) Anmeldenummer: 09014815.6
(22) Anmeldetag: 28.11.2009
(51) Int. Cl.: C07C 263/10, C07C 265/14

(54) **Verfahren zur Herstellung von Isocyanaten in der Gasphase**
Method for manufacturing isocyanates in the gas phase
Procédé de préparation d'isocyanates en phase gazeuse

(30) Priorität: 11.12.2008 DE 102008061686
(43) Veröffentlichungstag der Anmeldung: 16.06.2010
(73) Patentinhaber: Bayer MaterialScience AG, 51368 Leverkusen (DE)
(72) Erfinder: Pohl, Fritz, Dr., 25541 Brunsbüttel (DE); Biskup, Klaus, Dr., 25712 Buchholz (Dithm.) (DE); Bruns, Rainer, Dr., 51373 Leverkusen (DE); Lorenz, Wolfgang, Dr., 41540 Dormagen (DE); Steffens, Friedhelm, 51373 Leverkusen (DE); Michele, Volker, Dr., 51065 Köln (DE)

(56) Entgegenhaltungen:
- EP-A1- 1 275 639
- EP-A1- 1 449 826
- WO-A1-2008/055898

## Beschreibung

Die Erfindung betrifft ein Verfahren zur Herstellung von aromatischen Isocyanaten durch Umsetzung entsprechender primärer Amine mit Phosgen, **dadurch gekennzeichnet, dass** Phosgen und die primären aromatischen Amine oberhalb der Siedetemperatur der Amine in einem Reaktor enthaltend einen zur Strömungsrichtung im Wesentlichen rotationssymmetrischen Reaktionsraum umgesetzt werden, wobei die querschnittsgemittelte Strömungsgeschwindigkeit des Reaktionsgemischs längs der Achse des im Wesentlichen rotationssymmetrischen Reaktionsraumes in dem Abschnitt des Reaktionsraums, in dem der Umsatz der Amingruppen zu den Isocyanatgruppen zwischen 4 und 80 % liegt, maximal 8 m/sec beträgt und wobei die querschnittsgemittelte Strömungsgeschwindigkeit des Reaktionsgemisches längs der Achse des im Wesentlichen rotationssymmetrischen Reaktionsraumes in diesem Abschnitt des Reaktionsraumes stets unterhalb der querschnittsgemittelten Strömungsgeschwindigkeit am Anfang dieses Abschnittes liegt.

Isocyanate werden in großen Mengen hergestellt und dienen hauptsächlich als Ausgangsstoffe zur Herstellung von Polyurethanen. Ihre Herstellung erfolgt zumeist durch Umsetzung der entsprechenden Amine mit Phosgen. Eine Möglichkeit der Herstellung von Isocyanaten ist die Umsetzung der Amine mit dem Phosgen in der Gasphase. Diese üblicherweise als Gasphasenphosgenierung bezeichnete Verfahrensführung zeichnet sich dadurch aus, dass die Reaktionsbedingungen so gewählt werden, dass zumindest die Reaktionskomponenten Amin, Isocyanat und Phosgen, bevorzugt jedoch sämtliche Edukte, Produkte und Reaktionszwischenprodukte, bei den gewählten Bedingungen gasförmig sind. Vorteile der Gasphasenphosgenierung sind u.a. ein verringerter Phosgen-Hold-Up, die Vermeidung schwer phosgenierbarer Zwischenprodukte und erhöhte Reaktionsausbeuten. Die vorliegende Erfindung betrifft ausschließlich die Gasphasenphosgenierung.

Es sind aus dem Stand der Technik verschiedene Verfahren zur Herstellung von Isocyanaten durch Umsetzung von Aminen mit Phosgen in der Gasphase bekannt.

EP-A-289 840 beschreibt die Herstellung von Diisocyanaten durch Gasphasenphosgenierung, wobei die Herstellung erfindungsgemäß in einer turbulenten Strömung bei Temperaturen zwischen 200°C und 600°C in einem zylindrischen Raum ohne bewegte Teile stattfindet. Durch den Verzicht auf bewegte Teile wird die Gefahr eines Phosgenaustrittes reduziert. Gemäß der Lehre der EP-A-289 840 ist es für die Durchführbarkeit des in der EP-A-289 840 offenbarten Verfahrens wesentlich, dass die Abmessungen des Rohrreaktors und die Strömungsgeschwindigkeiten im Reaktionsraum so bemessen werden, dass in dem Reaktionsraum eine turbulente Strömung herrscht, die gemäß der Lehre von EP-A-289 840 durch eine Reynoldszahl von mindestens 2500, vorzugsweise mindestens 4700 charakterisiert wird. Nach der Lehre von EP-A-289 840 ist diese Turbulenz im Allgemeinen dann gewährleistet, wenn die gasförmigen Reaktionspartner den Reaktionsraum mit einer Strömungsgeschwindigkeit von mehr als 90 m/s durchlaufen. Durch die turbulente Strömung im zylindrischen Raum ( Rohr ) wird, wenn man von Fluidelementen in Wandnähe absieht, eine relative gute Strömungsgleichverteilung im Rohr und damit eine relativ enge Verweilzeitverteilung erreicht, die, wie in EP-A-570 799 beschrieben, zu einer Verringerung der Feststoffbildung führt. Nachteilig an dem in EP-A-289 840 offenbarten Verfahren ist, dass wegen der notwendigen hohen Strömungsgeschwindigkeiten die Realisierung der zur vollständigen Umsetzung der Amine notwendigen Verweilzeit, speziell bei Einsatz aromatischer Amine, nur in sehr langen Misch- und Reaktorrohren möglich ist.

EP-A-570 799 betrifft ein Verfahren zur Herstellung von aromatischen Diisocyanaten, **dadurch gekennzeichnet, dass** die Umsetzung des zugehörigen Diamins mit dem Phosgen in einem Rohrreaktor oberhalb der Siedetemperatur des Diamins innerhalb einer mittleren Kontaktzeit von 0,5 bis 5 Sekunden durchgeführt wird. Wie in der Schrift beschrieben ist, führen zu lange als auch zu kurze Reaktionszeiten zu einer unerwünschten Feststoffbildung. Es wird daher ein Verfahren offenbart, bei dem die mittlere Abweichung von der mittleren Kontaktzeit weniger als 6% beträgt. Die Einhaltung dieser Kontaktzeit wird dadurch erreicht, dass die Reaktion in einer Rohrströmung durchgeführt wird, die entweder durch eine Reynolds-Zahl von oberhalb 4.000 oder einer Bodenstein-Zahl von oberhalb 100 charakterisiert wird. Wird die erfindungsgemäße Rohrströmung durch eine Reynoldszahl oberhalb 4000 charakterisiert, ist nachteilig auch hier, dass wegen der notwendigen hohen Strömungsgeschwindigkeiten die Realisierung der zur vollständigen Umsetzung der Amine notwendigen Verweilzeit nur in sehr langen Misch- und Reaktorrohren möglich ist. Nach der Lehre der EP-A- 570 799 kann die erfindungsgemäße angenähert ideale Pfropfenströmung, charakterisiert durch eine Bodensteinzahl von mindestens 100, statt durch hochturbulente Strömungen auch durch Einbauten in das Reaktionsrohr realisiert werden, die der Ausbildung eines laminaren Strömungsprofils entgegen wirken und die Ausbildung einer ebenen Strömungsfront bewirken. Offenbart werden Einbauten in der Form eines dreidimensionalen feinmaschigen Drahtgitters oder einer Füllkörperfüllung. Nachteilig bei diesen Verfahrensvarianten ist, dass durch die Einbauten die Gefahr der Ausbildung von Ablagerungen in dem Reaktionsrohr erhöht wird, diese zu Verstopfungen und / oder Inhomogenitäten in der angestrebten angenähert idealen Pfropfenströmung führen können.

EP-A-699 657 beschreibt ein Verfahren zur Herstellung von aromatischen Diisocyanaten in der Gasphase, **dadurch gekennzeichnet, dass** die Umsetzung des zugehörigen Diamins mit dem Phosgen in einem zwei Zonen enthaltenden Reaktor stattfindet, wobei die erste Zone, die etwa 20% bis 80% des Gesamtreaktorvolumens ausmacht, ideal vermischt ist und die zweite Zone, die 80% bis 20% des Gesamtreaktorvolumens ausmacht, durch eine Kolbenströmung charakterisiert werden kann. Bevorzugt wird die zweite Reaktionszone als Rohrreaktor ausgeführt. Weil jedoch mindestens 20% des Reaktionsvolumens ideal rückvermischt sind, resultiert eine ungleichmäßige Verweilzeitverteilung, die zu einer unerwünschten erhöhten Feststoffbildung führen kann.

Die Optimierung des Einsatzes von Rohrreaktoren für die Gasphasenphosgenierung, wie er grundlegend in der EP-A- 570 799 unter Anwendung des Strahlmischerprinzips (Chemie-Ing.-Techn. 44 (1972) S.1055, Abb.10) offenbart wurde, ist Gegenstand zahlreicher Anmeldungen.

Nach der Lehre der EP-A-1 362 847 haben eine Vergleichmäßigung des über den Ringraum des Rohrreaktors zugeführten Eduktstromes und eine möglichst zentrale Zuführung beider Eduktströme in den Rohrreaktor einen großen positiven Einfluss auf die Stabilität der Reaktionszone und damit auf die Gasphasenreaktion insgesamt. Als Folge der stabileren Reaktionsführung nehmen die beobachteten Temperaturschwankungen deutlich ab, die ohne die offenbarten Maßnahmen zu beobachtende Asymmetrie in der Temperaturverteilung verschwindet praktisch vollständig. Nach der Lehre von EP-A-1 362 847 führen Temperaturschwankungen und Asymmetrien in der Temperaturverteilung zur Bildung von Nebenprodukten, die zu Anbackungen und Verstopfungen im Reaktor und damit zu einer Verkürzung der Standzeit der Reaktoren führen. Spezielle Hinweise zur Umsetzung des offenbarten Verfahrens in einem technischen Maßstab werden in der EP-A-1 362 847 jedoch nicht offenbart.

Wie in der EP-A-1 555 258 beschrieben, wird bei einer Vergrößerung der eingesetzten Rohrreaktoren auch eine Vergrößerung der Mischdüse, die häufig als Glattstrahldüse ausgebildet ist, notwendig. Mit der Vergrößerung des Durchmessers der Glattstrahldüse wird aber auch die Geschwindigkeit der Vermischung des Zentralstrahls durch den größeren erforderlichen Diffusionsweg verringert und die Gefahr von Rückvermischung erhöht, was wiederum zur Entstehung polymerer Verunreinigungen und damit fester Anbackungen im Reaktor führt. Nach der Lehre von EP-A-1 555 258 können die dargestellten Nachteile eliminiert werden, wenn der eine Eduktstrom über einen Ringspalt, der konzentrisch im Strom des anderen Eduktes angebracht ist, mit hoher Geschwindigkeit eingedüst wird. Dadurch werden der Diffusionsweg für die Vermischung klein und die Mischzeiten sehr kurz. Die Reaktion kann dann mit hoher Selektivität zum gewünschten Isocyanat ablaufen. Die Entstehung von polymeren Verunreinigungen und die Ausbildung von Anbackungen werden dadurch verringert. Wie in der EP-A-1 55 258 weiterhin offenbart, sind - bei vergleichbaren Geschwindigkeiten der Komponenten an der Mischstelle - bei dem erfindungsgemäßen Verfahren deutlich kürzere Reaktionsräume zur Erzielung der Maximaltemperatur im Reaktionssystem als bei dem Einsatz konventioneller Glattstrahldüsen erforderlich.

Gemäß der Lehre von EP-A-1 526129 hat eine Erhöhung der Turbulenz des Eduktstromes in der Zentraldüse einen positiven Einfluss auf die Durchmischung der Reaktanden und damit auf die Gasphasenreaktion insgesamt. Als Folge der besseren Durchmischung nimmt die Neigung zur Nebenproduktbildung ab und die erforderliche Verweilzeit und somit Reaktorbaulänge sinken deutlich. EP-A-1 526129 offenbart bei einem Einsatz einer Spiralwendel als Turbulenz erzeugendem Einbauelement in der Zentraldüse eine Verkürzung der Mischstrecke auf 42% der ursprünglichen Länge.

EP-A- 1 449 826 offenbart ein Verfahren zur Herstellung von Diisocyanaten durch Phosgenierung der entsprechenden Diamine, bei dem die dampfförmigen Diamine, gegebenenfalls verdünnt mit einem Inertgas oder mit den Dämpfen eines inerten Lösungsmittels, und Phosgen getrennt auf Temperaturen von 200°C bis 600°C erhitzt und in einem Rohrreaktor vermischt und zur Reaktion gebracht werden, **dadurch gekennzeichnet, dass** in dem Rohrreaktor eine Anzahl n ≥ 2 von parallel zur Achse des Rohrreaktors ausgerichtete Düsen angeordnet sind, wobei der die Diamine enthaltende Strom dem Rohrreaktor über die n Düsen zugeführt wird und der Phosgenstrom dem Rohrreaktor über den verbleibenden freien Raum zugeführt wird. Nach der Lehre der EP-A-1 449 826 sind Vorteile des erfindungsgemäßen Verfahrens u.a. eine Verkürzung der Mischzeiten gegenüber einer Einfachdüse (einzelnen Düse) mit gleicher Querschnittsfläche und damit einher gehend eine Verkürzung der erforderlichen Verweilzeit im Reaktor (Investitionskostenvorteil).

Eine Weiterentwicklung des Einsatzes von Rohrreaktoren für die Gasphasenphosgenierung, wie er grundlegend in der EP-A- 570 799 unter Anwendung des Strahlmischerprinzips (Chemie-Ing.-Techn. 44 (1972) S.1055, Abb.10) offenbart wurde, ist Gegenstand der WO2007/028715**.** WO2007/028715 offenbart ein Verfahren zur Herstellung von Isocyanaten durch Phosgenierung der entsprechenden Amine in der Gasphase in einem Reaktor, **dadurch gekennzeichnet, dass** der zum Einsatz kommende Reaktor eine Mischeinrichtung und einen Reaktionsraum aufweist. Gemäß der Lehre von WO2007/028715 umfasst der Reaktionsraum im vorderen Bereich den Mischraum, in dem überwiegend die Vermischung der gasförmigen Edukte Phosgen und Amin, gegebenenfalls vermischt mit Inertmedium, stattfindet, was in der Regel begleitet ist vom Einsetzen der Reaktion. Gemäß der Lehre von WO2007/028715 findet im hinteren Teil des Reaktionsraumes dann im Wesentlichen nur noch die Reaktion und höchstens untergeordnet die Vermischung statt. Bevorzugt werden in dem in WO2007/028715 offenbarten Verfahren zur Strömungsrichtung rotationssymmetrische Reaktionsräume eingesetzt, die konstruktiv im Wesentlichen in bis zu vier Längenabschnitte entlang der Längsachse des Reaktors im Verlauf der Strömung zerlegt werden können, wobei sich die Längenabschnitte in der Größe der durchströmten Querschnittsfläche unterscheiden. Nachteilig bei dem offenbarten Verfahren ist die hohe Strömungsgeschwindigkeit von bevorzugt 10 bis 300 m/s, besonders bevorzugt 40 bis 230, ganz besonders bevorzugt 50 bis 200, insbesondere mehr als 150 bis 190 und speziell 160 bis 180 m/s, mit der das gasförmige Reaktionsgemisch den Reaktionsraum durchläuft. Wie bereits in EP-A-570 799 beschrieben, ist auf Grund der hohen Strömungsgeschwindigkeiten die Realisierung der zur vollständigen Umsetzung der Amine notwendigen Verweilzeit, speziell bei Einsatz aromatischer primärer Amine, nur in sehr langen Reaktorrohren möglich. Nachteilig ist ebenfalls, dass die Veränderungen in der durchströmten Querschnittsfläche des Reaktionsraumes durch einen in einem Rohrreaktor befindlichen Volumenkörper erzeugt werden, die Umsetzung des offenbarten Reaktoraufbaus in einem technischen Maßstab somit konstruktiv aufwendig ist. Weiterhin ist nachteilig an in Rohrreaktoren befindlichen Volumenkörpern, dass diese wie auch die Einbauten nach der der Lehre von EP-A-570 799 die Gefahr der Ausbildung von Ablagerungen in dem Reaktionsrohr erhöhen, die zu Verstopfungen und damit zu einer verkürzten Standzeit der Reaktoren führen.

WO2008/055898 offenbart ein Verfahren zur Herstellung von Isocyanaten durch Phosgenierung der entsprechenden Amine in der Gasphase in einem Reaktor, **dadurch gekennzeichnet, dass** analog zu WO2007/028715 der zum Einsatz kommende Reaktor eine Mischeinrichtung und einen Reaktionsraum aufweist, der rotationssymmetrische Reaktionsraum konstruktiv im wesentlichen in bis zu vier Längenabschnitte entlang der Längsachse des Reaktors im Verlauf der Strömung zerlegt werden kann, wobei sich die Längenabschnitte in der Größe der durchströmten Querschnittsfläche unterscheiden. Gegenüber WO2007/028715 werden die Veränderungen der durchströmten Querschnittsflächen jedoch nicht durch einen in einen Rohrreaktor installierten Volumenkörper, sondern durch eine entsprechende Erweiterung bzw. Einschnürung der Reaktoraußenwandung erreicht. Nachteilig bei dem offenbarten Verfahren ist die hohe Strömungsgeschwindigkeit von bevorzugt 10 bis 300 m/s, besonders bevorzugt 40 bis 230, ganz besonders bevorzugt 50 bis 200, insbesondere mehr als 150 bis 190 und speziell 160 bis 180 m/s, mit der das gasförmige Reaktionsgemisch den Reaktionsraum durchläuft, wobei nach der Lehre von WO2008/055898 in Abschnitten gleicher oder zunehmender Fläche diese so zu wählen ist, dass die mittlere Geschwindigkeit des Reaktionsgemischs im Allgemeinen größer als 60 m/s ist. Zwar vermeidet das in WO2008/055 898 offenbarte Verfahren im Reaktionsraum befindliche Volumenkörper und reduziert damit gegenüber dem in WO 2007/028 715 offenbarten Verfahren die Gefahr der Ausbildung von Ablagerungen, es bleibt aber der Nachteil der hohen Strömungsgeschwindigkeit, denn auf Grund der hohen Strömungsgeschwindigkeiten ist die Realisierung der zur vollständigen Umsetzung der Amine notwendigen Verweilzeit, speziell bei Einsatz aromatischer primärer Amine, nur in sehr langen Reaktionsräumen möglich.

EP-A-1 275 639 offenbart als mögliche Verfahrensvariante zur Herstellung von Isocyanaten durch Phosgenierung der entsprechenden Amine mit Phosgen in der Gasphase ebenfalls den Einsatz eines Reaktors, bei dem der Reaktionsraum in Strömungsrichtung hinter der Vermischung der beiden Edukte eine Erweiterung der durchströmten Querschnittsfläche aufweist. Nach der Lehre von EP-A-1 275 639 kann diese Erweiterung der von dem Reaktionsgemisch durchströmten Querschnittsfläche schlagartig erfolgen, kann der Reaktionsraum der in dem offenbarten Verfahren eingesetzten Reaktoren auch eine kaskadenförmige und/oder kontinuierliche Veränderung der durchströmten Querschnittsfläche aufweisen. Nach der Lehre von EP-A-1 275 639 kann bei Einsatz einer kaskadenförmigen und/oder kontinuierlichen Veränderung der Geschwindigkeitsverlauf der Reaktionsmischung entlang der Achse des Reaktors eingestellt werden. Gemäß EP-A-1 275 639 führt eine Verengung des Querschnitts oder vorzugsweise eine leichte Erweiterung bis auf das doppelte, vorzugsweise bis auf das 1,5 fache des Anfangsquerschnittes wegen der Volumenvergrößerung während der Reaktion zu einer Beschleunigung der Strömung, was die Strömung stabilisiert und der Gefahr von Rückströmungen entgegenwirkt. Durch eine geeignet gewählte Erweiterung der Querschnittsfläche kann die Strömungsgeschwindigkeit der Reaktionsmischung über die Länge des Reaktors gerade konstant gehalten werden. Dadurch erhöht sich die zur Verfügung stehende Reaktionszeit bei gleich bleibender Länge des Reaktors.

Überraschenderweise wurde nun gefunden, dass zur Herstellung von aromatischen Isocyanaten durch Phosgenierung der entsprechenden Amine mit Phosgen in der Gasphase insbesondere solche Reaktoren mit einem zur Strömungsrichtung im Wesentlichen rotationssymmetrischen Reaktionsraum zum Einsatz kommen können, bei denen die querschnittsgemittelte Strömungsgeschwindigkeit des Reaktionsgemisches längs der Achse des im Wesentlichen rotationssymmetrischen Reaktionsraumes in dem Abschnitt des Reaktionsraums, in dem der Umsatz der Amingruppen zu den Isocyanatgruppen zwischen 4 und 80 % liegt, maximal 8 m/s beträgt und dabei gleichzeitig die querschnittsgemittelte Strömungsgeschwindigkeit des Reaktionsgemisches in diesem Abschnitt des Reaktionsraumes (d.h. im Bereich eines Umsatzes der Amingruppen zu den Isocyanatgruppen von 4% bis 80%) stets unterhalb der querschnittsgemittelten Strömungsgeschwindigkeit am Anfang dieses Abschnittes liegt. Der Einsatz der erfindungsgemäßen Reaktoren in Kombination mit den erfindungsgemäßen Reaktionsbedingungen ist insofern besonders überraschend, dass trotz der fehlenden Stabilisierung der Strömung des Reaktionsgemisches durch eine hohe Strömungsgeschwindigkeit sowie trotz der fehlenden Stabilisierung der Strömung des Reaktionsgemisches durch eine konstante oder sich beschleunigende Strömungsgeschwindigkeit des Reaktionsgemisches hohe Ausbeuten bei der Umsetzung der aromatischen Amine mit Phosgen zu den entsprechenden aromatischen Isocyanaten bei gleichzeitig langen Standzeiten der Reaktoren erreicht werden. Dies war in dieser Form nicht vorauszusehen. Der Einsatz von Reaktoren enthaltend zur Strömungsrichtung im Wesentlichen rotationssymmetrische Reaktionsräume mit den erfindungsgemäßen Reaktionsbedingungen führt zu einer für eine Umsetzung des Verfahrens in einen technischen Maßstab besonders vorteilhaften geringen Baulänge der Reaktoren.

Gegenstand der Erfmdung ist daher ein Verfahren zur Herstellung von aromatischen Isocyanaten durch Umsetzung von aromatischen primären Aminen mit Phosgen in der Gasphase, dadurch gekennzeichnet, dass Phosgen und die primären aromatischen Amine oberhalb der Siedetemperatur der Amine in einem Reaktor enthaltend einen zur Strömungsrichtung im Wesentlichen rotationssymmetrischen Reaktionsraum umgesetzt werden, wobei
a) die querschnittsgemittelte Strömungsgeschwindigkeit des Reaktionsgemisches längs der Achse des im Wesentlichen rotationssymmetrischen Reaktionsraumes in dem Abschnitt des Reaktionsraums, in dem der Umsatz der Amingruppen zu den Isocyanatgruppen zwischen 4 und 80 % liegt, maximal 8 m/s, bevorzugt 0,5 - 8 m/s, ganz besonders 1 - 6,5 m/s, beträgt und
b) die querschnittsgemittelte Strömungsgeschwindigkeit des Reaktionsgemisches längs der Achse des im Wesentlichen rotationssymmetrischen Reaktionsraumes in dem Abschnitt des Reaktionsraums, in dem der Umsatz der Amingruppen zu den Isocyanatgruppen zwischen 4 und 80 % liegt, stets unterhalb der querschnittsgemittelten Strömungsgeschwindigkeit am Anfang dieses Abschnittes liegt.

Die in a) und b) spezifizierten Geschwindigkeitsbedingungen sind wichtig in dem Bereich des Umsatzes der Amingruppen zu den Isocyanatgruppen von 4% bis 80 % (d.h. in dem Bereich des Reaktionsraums, in dem der Umsatz der Amingruppen zu den Isocyanatgruppen im Bereich von 4% bis 80% liegt), weil erst mit Erreichen eines Mindestumsatzes der Amingruppen zu den Isocyanatgruppen die Enthalpie der Umsetzung sowie die Volumenzunahme des Reaktionsgemisches auf Grund der Umsetzung einen signifikanten Einfluss auf die Strömung im Reaktionsraum nehmen und dann die Strömung im Reaktionsraum durch die wesentlichen Kenngrößen der Umsetzung ausgebildet wird. Dies ist ab einem Umsatz der Amingruppen zu den Isocyanatgruppen von 4%, bevorzugt sogar schon bei kleineren Umsätzen, weitgehend gegeben. Natürlich wird das erfindungsgemäße Verfahren vorteilhaft in gleicher Weise auch schon bis zum Erreichen der genannten Untergrenze von 4% des Umsatzes der Amingruppen zu den Isocyanatgruppen und damit bevorzugt im gesamten Bereich des Umsatzes der Amingruppen zu den Isocyanatgruppen bis 80% betrieben.

Aromatische Isocyanate im Sinn der vorliegenden Erfmdung sind solche, die mindestens eine an mindestens ein aromatisches Ringsystem gebundene Isocyanatgruppe aufweisen.

Gemäß dem erfmdungsgemäßen Verfahren erfolgt die Umsetzung von Phosgen mit aromatischen primären Aminen in der Gasphase. Unter Umsetzung in der Gasphase ist dabei zu verstehen, dass die Reaktionsbedingungen so gewählt werden, dass die Edukte, Reaktionszwischenprodukte und Produkte sowie gegebenenfalls zudosierte inerte Verbindungen im Verlauf der Umsetzung während des Durchgangs durch den Reaktionsraum überwiegend, insbesondere zu ≥ 95 Gew.-%, bevorzugt zu ≥ 98 Gew.-%, besonders bevorzugt ≥ 99 Gew.-%, ganz besonders bevorzugt ≥ 99,8 Gew.-% und speziell zu ≥ 99,9 Gew.-%, jeweils bezogen auf das Gewicht des Reaktionsgemisches, in der Gasphase verbleiben. Zwischenprodukte sind dabei beispielsweise bei Einsatz von Diaminen gebildete Monoamino-monocarbamoylchloride, Dicarbamoylchloride, Monoaminomonoisocyanate und Monoisocyanato- monocarbamoylchloride sowie die Hydrochloride der jeweiligen Aminoverbindungen.

Die erfindungsgemäße Umsetzung von Phosgen mit aromatischen primären Aminen erfolgt in zumindest einem Reaktionsraum, der im allgemeinen in einem Reaktor angeordnet ist, d.h. unter Reaktionsraum wird der Raum verstanden, in dem die Umsetzung der Edukte und Zwischenprodukte erfolgt, unter Reaktor wird die technische Vorrichtung verstanden, die den Reaktionsraum enthält. Dem Reaktionsraum werden im Allgemeinen die Edukte, gegebenenfalls verdünnt mit Inerten, über zumindest eine Mischeinrichtung zugeführt.

Bevorzugt umfasst der erfindungsgemäße Reaktionsraum im vorderen Bereich zumindest einen Mischraum, in dem überwiegend die Vermischung der gasförmig eintretenden Komponenten Phosgen und Amin, gegebenenfalls vermischt mit einem oder mehreren Inertmedien, stattfindet, was in der Regel begleitet ist vom Einsetzen der Umsetzung. Zu Unterscheidungszwecken wird als Mischraum der Bereich des Reaktionsraumes bezeichnet, in dem der Umsatz der eingesetzten Amingruppen zu den Isocyanatgruppen weniger als 4 % ist.

Als Umsatz der Amingruppen zu den Isocyanatgruppen im Sinn der vorliegenden Erfindung ist der Verbrauch an Amingruppen unter Bildung von Isocyanatgruppen zu verstehen. Dieser Umsatz lässt sich durch Probennahme längs des Reaktors und sofortiger Analyse der dem jeweiligen Reaktionsraumsegment entnommenen Probe mittels einer FT-IR-Technik unter Auswertung der NCO-Bande im Bereich von 2270 cm⁻¹ unmittelbar verfolgen, indem die gemessene Isocyanatgruppenkonzentration mit der bei einem vollständigen Umsatz der Amingruppen zu den Isocyanatgruppen zu erwartenden Isocyanatgruppenkonzentration in Relation gesetzt wird. Bevorzugt erfolgt die Probennahme über eine längs des Querschnitts des Reaktionsraumes verschiebbare Sonde und durchströmt das kontinuierlich dem Reaktionsraum entnommene Messgas dann möglichst verzögerungsfrei die Messzelle einer nach dem Stand der Technik eingesetzten FT-IR-Technik. Der Umsatz an Amingruppen zu den Isocyanatgruppen ist somit das Verhältnis der Konzentration der vorhandenen Isocyanatgruppen zu der theoretisch möglichen Konzentration an Isocyanatgruppen, wenn alle Amingruppen zu Isocyanatgruppen umgesetzt wären.

Als die querschnittsgemittelte Strömungsgeschwindigkeit des Reaktionsgemisches längs der Achse des zur Strömungsrichtung im Wesentlichen rotationssymmetrischen Reaktionsraumes ist im Sinne der Erfindung der Quotient aus über die Strömungsquerschnittsfläche aufintegriertem Volumenstrom und der Strömungsquerschnittsfläche zu verstehen.

"Rotationssymmetrisch" im Sinne der Erfindung bedeutet in Übereinstimmung mit dem Stand der Technik, siehe z.B. WO 2007/028 751 A1, S. 3, Z. 28 ff, dass ein Körper oder Raum, hier der Reaktionsraum, bei Drehung um die Rotationsachse eine Drehsymmetrie aufweist. Dabei kann es sich beispielsweise um eine zweizählige Drehachse C₂, um eine dreizählige C₃ oder vierzählige Drehachse C₄ handeln oder bevorzugt um eine vollständige Drehsymmetrie (C∝). So besitzt beispielsweise eine von einer Ellipse berandete Fläche eine zweizählige Drehachse. Als weiteres Beispiel besitzt eine von einem Kreis berandete Fläche eine vollständige Drehsymmetrie.

Nicht bevorzugt, aber prinzipiell auch möglich sind Reaktionsräume, die ovale oder aus beliebigen geschlossenen ebenen Polygonen zusammengesetzte Strömungsquerschnitte aufweisen.

"Zur Strömungsrichtung im Wesentlichen rotationssymmetrischer Reaktionsraum" im Sinne der Erfmdung bedeutet, dass sich der im Wesentlichen rotationssymmetrische Reaktionsraum von seinen Eigenschaften her ganz überwiegend wie ein rotationssymmetrischer Reaktionsraum verhält. Bevorzugt weichen dabei die Querschnittsflächen des Reaktionsraums, z.B. durch fertigungsbedingte Toleranzen, weniger als 10%, bevorzugt weniger als 5%, ganz besonders bevorzugt weniger als 2%, von der zugrundeliegenden mathematischen Rotationssymmetrie ab. Bevorzugt ist der im Wesentlichen rotationssymmetrische Reaktionsraum tatsächlich rotationssymmetrisch. Besonders bevorzugt handelt es sich dabei um einen Rohrreaktor mit einer sich, ggf. auch nur abschnittsweise, in Strömungsrichtung erweiternden, gleichbleibenden und/oder abnehmenden durchströmten Querschnittsfläche.

In einer bevorzugten Ausführung betrifft die Erfmdung ein Verfahren zur Herstellung von aromatischen Isocyanaten durch Umsetzung von aromatischen primären Aminen mit Phosgen in der Gasphase, **dadurch gekennzeichnet, dass** Phosgen und die primären aromatischen Amine oberhalb der Siedetemperatur der Amine in einem Reaktor enthaltend einen zur Strömungsrichtung im Wesentlichen rotationssymmetrischen Reaktionsraum umgesetzt werden, wobei
a) die querschnittsgemittelte Strömungsgeschwindigkeit des Reaktionsgemisches längs der Achse des im Wesentlichen rotationssymmetrischen Reaktionsraumes in dem Abschnitt des Reaktionsraums, in dem der Umsatz der Amingruppen zu den Isocyanatgruppen zwischen 4 und 90 %, besonders bevorzugt zwischen 4 und 99%, ganz besonders bevorzugt zwischen 4 und 99,5 % liegt, maximal 8 m/s, bevorzugt 0,5 - 8 m/s, ganz besonders bevorzugt 1 - 6,5 m/s, beträgt und
b) die querschnittsgemittelte Strömungsgeschwindigkeit des Reaktionsgemisches längs der Achse des im Wesentlichen rotationssymmetrischen Reaktionsraumes in dem Abschnitt des Reaktionsraums, in dem der Umsatz der Amingruppen zu den Isocyanatgruppen zwischen 4 und 90 %, besonders bevorzugt zwischen 4 und 99%, ganz besonders bevorzugt zwischen 4 und 99,5 % liegt, stets unterhalb der querschnittsgemittelten Strömungsgeschwindigkeit am Anfang dieses Abschnittes liegt.

Bevorzugt beträgt die querschnittsgemittelte Strömungsgeschwindigkeit des Reaktionsgemisches auch schon im Bereich des Umsatzes der Amingruppen zu den Isocyanatgruppen von 0 bis 4 % max. 8 m/s. Ebenso beträgt die querschnittsgemittelte Strömungsgeschwindigkeit des Reaktionsgemisches bevorzugt auch im Bereich des Umsatzes der Amingruppen zu den Isocyanatgruppen von > 99,5% maximal 8 m/s.

In einer weiteren bevorzugten Ausführungsform des erfindungsgemäßen Verfahrens wird die Absenkung der querschnittsgemittelten Strömungsgeschwindigkeit des Reaktionsgemischs längs der Achse des rotationssymmetrischen Reaktionsraumes durch mindestens eine Erweiterung, bevorzugt eine konusförmige Erweiterung, der durchströmten Querschnittsfläche des Reaktionsraumes im erfindungsgemäßen Bereich erreicht. Bevorzugt weist der Reaktionsraum im Aufweitungsbereich stets Aufweitungshalbwinkel, d.h. Winkel zwischen Reaktionsraumwandung und Reaktionsraumachse, von im allgemeinen ≤ 6°, bevorzugt ≤ 3,5°, besonders bevorzugt ≤ 2°, auf.

In einer weiteren bevorzugten Ausführungsform des erfindungsgemäßen Verfahrens weist der Reaktor im Anschluss an den Reaktionsraum, in dem nach der Mischung der Edukte ein Umsatz der Amingruppen zu den Isocyanatgruppen von 80%, bevorzugt 90%, besonders bevorzugt 99%, ganz besonders bevorzugt 99,5% erzielt wird, einen rotationssymmetrischen Reaktionsraum mit konstanter und/oder erweiterter durchströmter Querschnittsfläche auf.

In einer weiteren bevorzugten Ausführungsform weist der Reaktionsraum, der dem Abschnitt, in dem nach der Mischung der Edukte ein Umsatz der Amingruppen zu den Isocyanatgruppen von 80%, bevorzugt 90%, besonders bevorzugt 99%, ganz besonders bevorzugt 99,5% erzielt wird, nachgeschaltet ist, zumindest eine Zone auf, in der zum Abbruch der Umsetzung des Amins mit dem Phosgen zum Isocyanat zumindest eine Flüssigkeit eingedüst wird.

In diesem Zusammenhang ist festzuhalten, dass die Gesamtreaktion der Amingruppen mit dem Phosgen zu den Isocyanatgruppen nicht allein den Verbrauch des eingesetzten Amins, sondern ebenfalls die Umsetzung der bei dem Verbrauch des eingesetzten Amins entstehenden Zwischenprodukte zu dem Isocyanat umfasst. Beispiele möglicher Zwischenprodukte sind bei dem Einsatz eines Diamins Monoamino-monocarbamoylchloride, Dicarbamoylchloride, Monoaminomonoisocyanate und Monoisocyanato- monocarbamoylchloride sowie die Hydrochloride der jeweiligen Aminoverbindungen. Bei der Überführung einer Gasphasenphosgenierung in einen technischen Maßstab bestimmen die für die Umsetzung der Zwischenprodukte zu dem Isocyanat notwendigen Reaktionsbedingungen, beispielsweise die zur Umsetzung notwendigen Reaktionszeiten, nicht unwesentlich die Auslegung der technischen Apparate.

Für das erfindungsgemäße Verfahren können primäre aromatische Amine verwendet werden. Bevorzugt werden primäre aromatische Amine eingesetzt, die im Wesentlichen ohne Zersetzung in die Gasphase überführt werden können.

Beispiele für bevorzugte aromatische Amine sind Toluylendiamin (TDA), insbesondere 2,4- TDA und 2,6-TDA und Gemische daraus, Diaminobenzol, Naphthyldiamin (NDA) und 2,2'-, 2,4'-oder 4,4'-Methylendiphenyldiamin (MDA) oder Isomerengemische davon. Besonders bevorzugt ist Toluylendiamin (TDA), insbesondere 2,4-TDA und 2,6-TDA und Gemische daraus.

Die Ausgangsamine werden vor der Durchführung des erfindungsgemäßen Verfahrens in der Regel verdampft und auf 200°C bis 600°C, bevorzugt 200°C bis 500°C, besonders bevorzugt 250°C bis 450°C erhitzt und gegebenenfalls verdünnt mit einem Inertgas wie N₂, He, Ar oder mit den Dämpfen eines inerten Lösungsmittels, z.B. aromatischen Kohlenwasserstoffen ggf. mit Halogensubstitution, wie z.B. Chlorbenzol oder o-Dichlorbenzol, dem Reaktionsraum zugeführt.

Die Verdampfung der Ausgangsamine kann in allen bekannten Verdampfungsapparaturen erfolgen, bevorzugt werden Verdampfungssysteme, bei denen ein kleiner Arbeitsinhalt mit einer hohen Umwälzleistung über einen Fallfilmverdampfer geführt, dabei zur Minimierung der thermischen Belastung der Ausgangsamine der Verdampfungsvorgang - wie oben ausgeführt- ggf. unter Zuspeisung von Inertgas und / oder Dämpfen eines inerten Lösungsmittels unterstützt wird.

In einer besonders bevorzugten Ausführungsform werden Verdampfungssysteme eingesetzt, bei denen ein kleiner Arbeitsinhalt über zumindest einen Mikrowärmeaustauscher oder Mikroverdampfer umgewälzt wird. Der Einsatz entsprechender Wärmeaustauscher für die Verdampfung von Aminen wird z.B. in EP-A-1 754 698 offenbart. Bevorzugt werden in dem erfindungsgemäßen Verfahren die in den Absätzen (0007) bis (0008) und (0017) bis (0039) der EP-A-1 754 689 offenbarten Apparate eingesetzt.

Die dampfförmigen Amine können noch Anteile an unverdampften Tröpfchen an Aminen (Aerosol) enthalten. Bevorzugt enthalten die dampfförmigen Amine jedoch im wesentlichen keine Tröpfchen an unverdampften Aminen, das heißt maximal 0,5 Gew.-% des Amins, besonders bevorzugt maximal 0,05 Gew.-% des Amins, bezogen auf das Gesamtgewicht des Amins, liegt in der Form von unverdampften Tröpfchen vor und der restliche Teil des Amins liegt dampfförmig vor. Ganz besonders bevorzugt enthalten die dampfförmigen Amine keine Tröpfchen an unverdampften Aminen. Bevorzugt wird nach der Verdampfung das dampfförmige Amin, ggf. mit Inertgasen oder inerten Lösungsmitteldämpfen verdünnt, über einen Nacherhitzer auf die gewünschte Einsatztemperatur gebracht.

Weiterhin bevorzugt erfolgt die Verdampfung und Überhitzung der Ausgangsamine mehrstufig, um unverdampfte Tröpfchen im dampfförmigen Aminstrom zu vermeiden. Insbesondere bevorzugt sind mehrstufige Verdampfungs- und Überhitzungsschritte, in denen zwischen den Verdampfungs- und Überhitzungssystemen Tröpfchenabscheider eingebaut sind und / oder die Verdampfungsapparaturen auch die Funktion eines Tröpfchenabscheiders besitzen. Geeignete Tröpfchenabscheider sind z.B. beschrieben in "Droplet Separation", A. Bürkholz, VCH Verlagsgesellschaft, Weinheim - New York - Basel - Cambridge, 1989. Besonders bevorzugt sind Tröpfchenabscheider die einen geringen Druckverlust verursachen. Ganz besonders bevorzugt wird das verdampfte Amin über zumindest einen Nacherhitzer auf die gewünschte Einsatztemperatur gebracht, der auch als Tröpfchenabscheider fungiert. Insbesondere bevorzugt hat dieser Nacherhitzer einen Flüssigkeitsablauf um die stetige Entleerung des Abscheiders zu gewährleisten. Nach Verlassen des in Strömungsrichtung letzten Überhitzers wird das auf seine Solltemperatur vorgeheizte dampfförmige Amin mit einer mittleren Verweilzeit von bevorzugt 0,01 bis 60s, ganz besonders bevorzugt von 0,01 bis 30s, insbesondere bevorzugt 0,01-15 s, dem Reaktor bzw. dessen Mischeinrichtung zur Umsetzung zugeführt, dabei wird über technische Maßnahmen, z.B. eine ausreichende Isolierung zur Vermeidung von Abstrahlungsverlusten, der Gefahr einer erneuten Tröpfchenbildung begegnet. Durch die Erzeugung eines im Wesentlichen tröpfchenfreien dampfförmigen Ausgangsaminstromes vor Eintritt in den Reaktor wird die Reaktorlaufzeit deutlich erhöht.

In einer bevorzugten Ausführungsform erfolgt die Zuführung des dampfförmigen Aminstromes auf den Reaktor bzw. dessen zumindest eine Mischeinrichtung druckverlustarm ohne Regeleinrichtung, eine geregelte Zuführung ist jedoch ebenfalls möglich. Möglich ist auch eine Aufteilung des Aminstromes in mehrere Teilströme, die dann, wie z.B. in EP-A-1 449 826 in den Absätzen (0019) bis (0022) beschrieben, einem Reaktionsraum oder, wie z.B. in WO 2008/055898 Seite 8/Zeile 25 bis Seite 15/Zeile 31 und insbesondere Seite 23/Zeilen 19 - 31 beschrieben, mehreren Mischeinrichtungen zugeführt werden. Bevorzugt erfolgt auch bei einem Split des dampfförmigen Aminstromes die Zuführung der Aminteilströme druckverlustarm ohne zusätzliche Regeleinrichtungen. Es ist aber auch eine getrennt geregelte Zuführung der Teilströme möglich.

Beim erfindungsgemäßen Verfahren ist es vorteilhaft, Phosgen im Überschuss bezüglich der umzusetzenden Amingruppen einzusetzen. Bevorzugt liegt ein molares Verhältnis von Phosgen zu Amingruppen von 1,1:1 bis 20:1, bevorzugt 1,2:1 bis 5:1 vor. Auch das Phosgen wird auf Temperaturen von 200°C bis 600°C erhitzt und gegebenenfalls verdünnt mit einem Inertgas wie N₂, He, Ar oder mit den Dämpfen eines inerten Lösungsmittels, z.B. aromatischen Kohlenwasserstoffen ohne oder mit Halogensubstitution, wie z.B. Chlorbenzol oder o-Dichlorbenzol, dem Reaktionsraum zugeführt.

In einer bevorzugten Ausführungsform erfolgt eine geregelte Zuführung des Phosgenstromes auf den Reaktor bzw. dessen zumindest eine Mischeinrichtung. Eine druckverlustarme Zuführung ohne Regeleinrichtung ist jedoch ebenfalls möglich. Möglich ist auch eine Aufteilung des Phosgenstromes in mehrere Teilströme, die dann, wie z.B. in WO 2008/055898 Seite 8/Zeile 25 bis Seite 15/Zeile 31 und insbesondere Seite 23/Zeilen 19 - 31 beschrieben, mehreren Mischeinrichtungen eines Reaktors zugeführt werden. Auch die Zuführung der Teilströme auf mehrere Reaktoren ist möglich. Bevorzugt erfolgt bei einem Split des Phosgenstromes eine getrennt geregelte Zuführung der Phosgenteilströme.

Das erfindungsgemäße Verfahren wird so durchgeführt, dass die getrennt aufgeheizten Reaktionspartner über zumindest eine Mischeinrichtung in wenigstens einen Reaktionsraum eingeführt, vermischt und unter Beachtung geeigneter Reaktionszeiten unter bevorzugt adiabater Reaktionsführung umgesetzt werden. Anschließend wird durch Abkühlung des Gasstromes das Isocyanat kondensiert, wobei die Abkühlung bis zu einer Temperatur oberhalb der Zersetzungstemperatur des entsprechenden Carbaminsäurechlorids, also beispielsweise Toluylen-monoisocyanat-monocarbaminsäurechlorid und/oder im Falle von TDA, erfolgt.

Die notwendige Verweilzeit zur Reaktion der Amingruppen mit dem Phosgen zu den Isocyanatgruppen liegt zwischen 0,05 und 15 Sekunden, in Abhängigkeit von der Art des eingesetzten Amins, der Starttemperatur, der adiabaten Temperaturerhöhung im Reaktionsraum, dem molaren Verhältnis von eingesetztem Amin und Phosgen, einer etwaigen Verdünnung der Reaktionspartner mit Inertgasen sowie dem gewählten Reaktionsdruck.

Wird für das jeweilige System (eingesetztes Amin, Starttemperatur, adiabate Temperaturerhöhung, molares Verhältnis der Reaktanden, Verdünnungsgas, Reaktionsdruck ) eine einmal ermittelte Mindestverweilzeit für die vollständige Reaktion um weniger als 20%, vorzugsweise weniger als 10% überschritten, kann die Bildung von Folge-Reaktionsprodukten wie Isocyanuraten und Carbodiimiden weitgehend vermieden werden.

Innerhalb dieses für chemische Reaktionen sehr engen Kontaktzeitspektrums muss sowohl die möglichst homogene Vermischung der Reaktionspartner als auch die weitere Reaktion erfolgen. Dabei erfolgt die weitere Reaktion bevorzugt ohne Rückvermischung, die eine Verbreiterung des Kontaktzeitraums und damit eine vermehrte Bildung von unerwünschten Neben- und Folgeprodukten bewirken würde.

Bei der praktischen Durchführung des Verfahrens kann eine Abweichung von der mittleren Kontaktzeit durch die notwendige Zeit der Vermischung der Reaktionspartner erfolgen. Die Methoden zur Durchführung kurzer Mischzeiten sind im Prinzip bekannt. Geeignet sind beispielsweise Mischaggregate oder Mischräume mit bewegten oder statischen Mischorganen oder Düsen. Bevorzugt ist der Einsatz statischer Mischer in Mischräumen, wie er z.B. in EP-A-1 362 847, EP-A-1 526 129 oder EP-A- 1 555 258 beschrieben ist. Bevorzugt werden in dem erfindungsgemäßen Verfahren die in den Absätzen (0008) bis (0014) und (0023) bis (0026) der EP-A-1 362 847, die in den Absätzen (0008) bis (0013) und (0022) bis (0026) der EP-A-1 526 129 oder die in den Absätzen (0007) und (0024) bis (0025) der EP-A-1 555 258 offenbarten Apparate eingesetzt.

Besonders bevorzugt kommen Reaktoren mit im Wesentlichen rotationssymmetrischen Reaktionsräumen zum Einsatz, bei denen die gasförmigen Edukte, ggf. verdünnt mit Inerten, dem zumindest einen Mischraum nach dem Strahlmischerprinzip (Chemie-Ing. Techn. 44 (1972) S. 1055, Abb.10) zugeführt werden. Bevorzugt treten dabei die eingespeisten Stoffströme mit einem Geschwindigkeitsverhältnis von 2-20, besonders bevorzugt 3-15, ganz besonders bevorzugt 4-12, in den zumindest einen Mischraum der Reaktoren ein. Bevorzugt wird dabei dem zumindest einen Mischraum der Reaktoren das Amin, ggf. verdünnt mit Inerten, mit der höheren Strömungsgeschwindigkeit zugeführt.

Bevorzugt weisen weder der Reaktionsraum noch etwaige Mischaggregate oder Mischräume Heizflächen auf, die Anlass zu einer thermischen Belastung mit der Folge von Folgereaktionen wie Isocyanurat- oder Carbodiimidbildung geben können, oder Kühlflächen auf, die Anlass einer Kondensation mit der Folge von Ablagerungen geben können. Die Komponenten werden so, abgesehen von etwaigen Abstrahlungs- und Ableitungsverlusten, bevorzugt adiabat umgesetzt, dabei wird die adiabate Temperaturerhöhung im Mischaggregat und Reaktor bzw. Reaktor allein über die Temperaturen, Zusammensetzungen und relativen Dosierungen der Eduktströme sowie der Verweilzeit in den Mischaggregaten und den Reaktoren eingestellt.

Nach der erfolgten Phosgenierungsreaktion im Reaktionsraum wird das gasförmige Reaktionsgemisch, das bevorzugt wenigstens ein Isocyanat, Phosgen und Chlorwasserstoff umfasst, von dem gebildeten Isocyanat befreit. Dies kann beispielsweise dadurch erfolgen, indem das den Reaktionsraum kontinuierlich verlassende Gemisch, bevorzugt wenigstens ein Isocyanat, Phosgen und Chlorwasserstoff umfassend, nach Verlassen des Reaktionsraumes einer Kondensation in einem inerten Lösungsmittel unterzogen wird, wie sie bereits für andere Gasphasenphosgenierungen empfohlen wurde (EP-A-0 749 958).

Bevorzugt erfolgt die Kondensation jedoch dadurch, dass der in dem erfindungsgemäßen Verfahren eingesetzte Reaktionsraum zumindest eine Zone aufweist, in die zum Abbruch der Umsetzung der eingesetzten Amine und des Phosgens zu den entsprechenden Isocyanaten ein oder mehrere geeignete Flüssigkeitsströme ("Quench-Flüssigkeiten") eingesprüht werden. Dadurch kann, wie in EP-A-1 403 248 beschrieben, eine rasche Abkühlung der Gasgemische ohne den Einsatz kalter Flächen durchgeführt werden.

In einer besonders bevorzugten Form des erfmdungsgemäßen Verfahrens ist die zumindest eine Zone (Kühlzone) in eine Quenchstufe, wie sie z. B. in EP-A-1 403 248 offenbart wurde, integriert. In einer besonders bevorzugten Form kommen mehrere Kühlzonen zum Einsatz, erfolgen Integration und Betrieb dieser zumindest zwei Kühlzonen mit einer Quenchstufe. Dies ist hinsichtlich Aufbau und Betrieb in EP-A-1 935 875 offenbart.

Statt des integrierten Verbundes von der zumindest einen Kühlzone eines Reaktors mit einer Quenchstufe, wie sie in der EP-A-1 935 875 offenbart wurde, ist ebenfalls der entsprechende integrierte Verbund der Kühlzonen mehrerer Reaktoren mit einer Quenchstufe möglich. Bevorzugt ist jedoch der integrierte Verbund eines Reaktors mit zumindest einer Kühlzone mit einer Quenchstufe.

In einer bevorzugten Ausführungsform des erfindungsgemäßen Verfahrens beträgt die Durchsatzkapazität des eingesetzten Reaktors bei den erfindungsgemäß geforderten Reaktionsbedingungen > 1 t Amin / h, bevorzugt 2 - 50 t Amin / h, besonders bevorzugt 2-12 t Amin / h. Besonders bevorzugt gelten diese Werte für Toluylendiamin. Unter Durchsatzkapazität ist dabei zu verstehen, dass in dem Reaktor die genannte Durchsatzkapazität an Amin pro h umgesetzt werden kann.

Unabhängig von der Art der gewählten Abkühlung wird die Temperatur der zumindest einen Abkühlzone bevorzugt so gewählt, dass sie einerseits oberhalb der Zersetzungstemperatur des dem Isocyanat entsprechenden Carbamoylchlorids liegt und andererseits das Isocyanat und gegebenenfalls das in dem Amindampfstrom und / oder Phosgenstrom als Verdünnungsmittel mit verwendete Lösungsmittel weitestgehend kondensieren bzw. sich weitestgehend in dem Lösungsmittel lösen, während überschüssiges Phosgen, Chlorwasserstoff und ggf. als Verdünnungsmittel mit verwendetes Inertgas die Kondensations- bzw. Quenchstufe weitestgehend nicht kondensiert bzw. gelöst durchlaufen. Zur selektiven Gewinnung des Isocyanats aus dem gasförmig Reaktionsgemisch besonders gut geeignet sind bei einer Temperatur von 80 bis 200°C, vorzugsweise 80 bis 180 °C gehaltene Lösungsmittel wie beispielsweise Chlorbenzol und / oder Dichlorbenzol, oder in diesen Temperaturbereichen gehaltenes Isocyanat oder Mischungen des Isocyanats mit Chlorbenzol und / oder Dichlorbenzol. Es ist für den Fachmann aufgrund der physikalischen Daten bei gegebener Temperatur, Druck und Zusammensetzung einfach vorhersagbar, welcher Massenanteil des Isocyanates in der Quenche kondensiert bzw. diese nicht kondensiert durchläuft. Ebenso ist es einfach vorherzusagen, welcher Massenanteil des überschüssigen Phosgens, Chlorwasserstoffs und ggf. als Verdünnungsmittel verwendeten Inertgases die Quenche unkondensiert durchläuft bzw. sich in der Quencheflüssigkeit löst.

Die Erzeugung der für das erfindungsgemäße Verfahren bevorzugten Strömung des gasförmigen Reaktionsgemischs als Strömung ohne wesentliche Rückvermischung durch den Reaktionsraum wird durch ein Druckgefälle über den Reaktionsraum sichergestellt. Bevorzugt besteht das Druckgefälle zwischen den Eduktzuleitungen vor der Vermischung einerseits und dem Ausgang aus der Kondensations- bzw. Quenchstufe andererseits. Bevorzugt liegt der absolute Druck in den EduktZuleitungen vor der Vermischung bei 200 bis 3000 mbar und hinter der Kondensations- bzw. Quenchstufe bei 150 bis 2500 mbar. Wesentlich ist hierbei jedoch lediglich die Aufrechterhaltung eines Differenzdrucks von den Eduktzuleitungen über den Reaktionsraum bis hinter die Kondensations- bzw. Quenchstufe von bevorzugt mindestens 50 mbar zwecks Gewährleistung der genannten gerichteten Strömung und einer guten Vermischung der Edukte.

Das die Kondensations- bzw. Quenchstufe verlassende Gasgemisch wird bevorzugt in einer nachgeschalteten Gaswäsche mit einer geeigneten Waschflüssigkeit von restlichem Isocyanat befreit, bevorzugt anschließend in an sich bekannter Weise von überschüssigem Phosgen befreit. Dies kann mittels einer Kühlfalle, Absorption in einem inerten Lösungsmittel (z.B. Chlorbenzol oder Dichlorbenzol) oder durch Adsorption und Hydrolyse an Aktivkohle erfolgen. Das die Phosgenrückgewinnungsstufe durchlaufende Chlorwasserstoffgas kann in an sich bekannter Weise zur Rückgewinnung des zur Phosgensynthese erforderlichen Chlors recyclisiert werden. Die nach ihrem Einsatz zur Gaswäsche anfallende Waschflüssigkeit wird dann bevorzugt zumindest teilweise als Quench-Flüssigkeit zur Abkühlung des Gasgemisches in die entsprechende Zone des Reaktionraumes eingesetzt.

Die Reindarstellung der Isocyanate erfolgt bevorzugt anschließend durch destillative Aufarbeitung der Lösungen bzw. Gemische aus der Kondensations- bzw. Quenchstufe.

### Beispiele

Die Bestimmung des Umsatzes der Amingruppen zu den Isocyanatgruppen ist möglich durch Probennahme längs der eingesetzten Reaktoren und sofortiger Analyse der dem jeweiligen Reaktionsraumsegment entnommenen Probe mittels einer FT-IR-Technik unter Auswertung der NCO-Bande im Bereich von 2270 cm⁻¹. Dabei erfolgt die Probennahme in den Segmenten über eine längs des Querschnitts des Reaktionsraumsegmentes verschiebbare Sonde und durchströmt das kontinuierlich dem Reaktionsraum entnommene Messgas dann die Messzelle einer nach dem Stand der Technik eingesetzten FT-IR-Technik. Für die Bestimmung der Isocyanatgruppenkonzentration an der Meßstelle wird die Sonde radial über den Reaktionsraumquerschnitt an der Meßstelle verschoben, um entsprechende Messungen der lokalen Isocyanatgruppenkonzentration an mehreren Orten zu erhalten. Aus dem so erhaltenen Isocyanatgruppenkonzentrationsprofil wird eine querschnittsgemittelte Isocyanatgruppenkonzentration berechnet und in Folge als Quotient aus der querschnittsgemittelten Isocyanatgruppenkonzentration zu der bei einem vollständigen Umsatz der Amingruppen zu den Isocyanatgruppen sich ergebenden Isocyanatgruppenkonzentration der Umsatz der Amingruppen zu den Isocyanatgruppen an der Meßstelle bestimmt.

Die Bestimmung der querschnittsgemittelten Strömungsgeschwindigkeit ist möglich durch Einsatz eines Prandtlrohrs zur Messung des dynamischen Drucks, wie es z.B. in W. Bohl, "Technische Strömungslehre", 5. überarbeitete Auflage, Vogel-Buchverlag, Würzburg, 1982, S.217 f., beschrieben ist. Der mit Hilfe des Prandtlrohres gemessene lokale Druck wird unter Abschätzung der lokalen Gasdichte in eine lokale Strömungsgeschwindigkeit umgerechnet. Für die Bestimmung der querschnittsgemittelten Strömungsgeschwindigkeit an der Meßstelle wird das Prandtlrohr radial über den Reaktionsraumquerschnitt an der Meßstelle verschoben, um entsprechende Messungen der lokalen Geschwindigkeit an mehreren Orten zu erhalten. Aus dem so erhaltenen Geschwindigkeitsprofil wird durch eine numerische Integration der Gasvolumenstrom und in der Folge als Quotient aus über die Strömungsquerschnittsfläche aufintegriertem Volumenstrom und der Strömungsquerschnittsfläche die querschnittsgemittelte Strömungsgeschwindigkeit bestimmt.

Die im folgenden dargestellten Beispiele basieren auf einer strömungstechnischen Simulation auf Basis der Lösung der Navier-Stokes-Gleichungen, wie sie z.B. in A. Paschedag, "CFD in der Verfahrenstechnik", Wiley-VCH-Verlag, Weinheim, 2004 in ihren wesentlichen Zügen dargestellt ist. In diesem Grundmodell wurden kinetische Daten der Umsetzung von 2,4-TDA mit Phosgen eingesetzt.

### Beispiel 1 (nicht erfindungsgemäß):

2500 kg/h 2,4-Toluylendiamin (kurz TDA) werden verdampft und gasförmig mit einer Temperatur von 410°C einem zylindrischen Rohrreaktor (Durchmesser 380 mm) über eine auf der Reaktorachse angeordneten Düse (Durchmesser 70 mm) zugeleitet. Gleichzeitig werden parallel dazu 8097 kg/h gasförmiges Phosgen auf 390°C erwärmt und auf dem von der einen Düse freigelassenen Ringraum ebenfalls dem Rohrreaktor zugeführt. Die Ströme werden in dem Rohrreaktor bei einem Betriebsdruck von 1400 mbar abs. gemischt, wobei zeitgleich die Umsetzung der Amingruppen zu den Isocyanatgruppen einsetzt. 630 mm stromab der Düsenmündung ist ein Umsatz der Amingruppen zu den Isocyanatgruppen von 12% erreicht, die querschnittsgemittelte Strömungsgeschwindigkeit beginnt dort aufgrund der Volumenexpansion infolge Reaktionsstöchiometrie und - exothermie signifikant zu steigen. Es zeigt sich, dass die querschnittsgemittelte Strömungsgeschwindigkeit ab einem Umsatz der Amingruppen zu den Isocyanatgruppen von 12%, d.h. nach 630 mm Länge ab Düsenmündung, stets höher ist als bei dem Umsatz der Amingruppen zu den Isocyanatgruppen von 12%, d.h. bei der Länge von 630 mm ( dort 9,9 m/s), es wird ein Endwert von 16,3 m/s am Reaktoraustritt erreicht. Bis zum weitestgehend vollständigen Abschluss der Umsetzung des 2,4-Toluylendiamins und seiner Folgeprodukte mit dem Phosgen ist bei einer 2,4-Toluylendiisocyanatausbeute von > 99,5% eine minimale Reaktionsraumlänge ab der Düsenmündung von 19,9 m erforderlich.

### Beispiel 2 (nicht erfindungsgemäß):

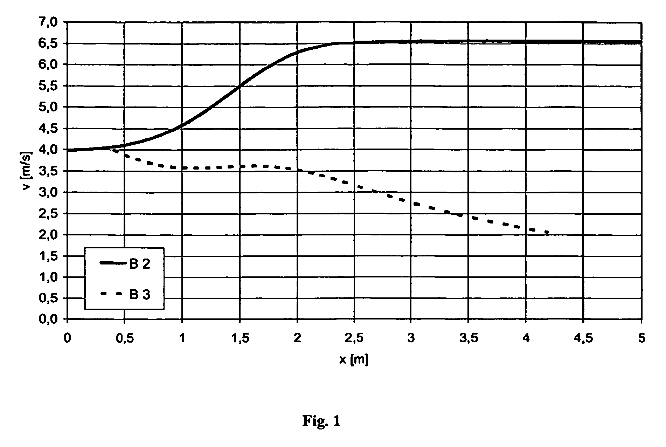

2500 kg/h 2,4-Toluylendiamin (kurz TDA) werden verdampft und gasförmig mit einer Temperatur von 410°C einem zylindrischen Rohrreaktor (Durchmesser 600 mm) über eine auf der Reaktorachse angeordnete Düse (Durchmesser 110 mm) zugeleitet. Gleichzeitig werden parallel dazu 8097 kg/h gasförmiges Phosgen auf 390°C erwärmt und auf dem von der einen Düse freigelassenen Ringraum ebenfalls dem Rohrreaktor zugeführt. Die Ströme werden in dem Rohrreaktor bei einem Betriebsdruck von 1400 mbar abs. gemischt, wobei zeitgleich die Umsetzung der Amingruppen zu den Isocyanatgruppen einsetzt. 350 mm stromab der Düsenmündung ist ein Umsatz der Amingruppen zu den Isocyanatgruppen von 4% erreicht, die querschnittsgemittelte Strömungsgeschwindigkeit beginnt dort aufgrund der Volumenexpansion infolge Reaktionsstöchiometrie und -exothermie signifikant zu steigen. Der Verlauf der querschnittsgemittelten Strömungsgeschwindigkeit gegenüber der Länge des Rohrreaktors ist in Fig. 1 dargestellt. Es zeigt sich, dass die querschnittsgemittelte Strömungsgeschwindigkeit ab einem Umsatz der Amingruppen zu den Isocyanatgruppen von 4%, d.h. nach 350 mm Länge ab Düsenmündung, stets höher ist als bei dem Umsatz der Amingruppen zu den Isocyanatgruppen von 4%, d.h. bei der Länge von 350 mm. Bis zum weitestgehend vollständigen Abschluss der Umsetzung des 2,4-Toluylendiamins und seiner Folgeprodukte mit dem Phosgen ist bei einer 2,4-Toluylendiisocyanatausbeute von > 99,5% eine minimale Reaktionsraumlänge ab der Düsenmündung von 8 m erforderlich.

### Beispiel 3 (erfindungsgemäß):

2500 kg/h 2,4-Toluylendiamin (kurz TDA) werden verdampft und gasförmig mit einer Temperatur von 410°C einem zylindrisch-konischen Rohrreaktor über eine auf der Reaktorachse angeordnete Düse (Durchmesser 110 mm) zugeleitet. Gleichzeitig werden parallel dazu 8097 kg/h gasförmiges Phosgen auf 390°C erwärmt und auf dem von der einen Düse freigelassenen Ringraum ebenfalls dem Rohrreaktor zugeführt. Der Reaktor hat im Bereich der Eduktzuführung einen Durchmesser von 600 mm; dieser Durchmesser wird bis zu einer axialen Position 350 mm stromab der Düsenmündung beibehalten, danach folgt ein Abschnitt mit einer konischen Aufweitung (Aufweitungshalbwinkel, d.h. Winkel zwischen Reaktorwand und Reaktorachse, 3,5°). Die Ströme werden in dem Rohrreaktor bei einem Betriebsdruck von 1400 mbar abs. gemischt, wobei zeitgleich die Umsetzung der Amingruppen zu den Isocyanatgruppen einsetzt. 350 mm stromab der Düsenmündung ist ein Umsatz der Amingruppen zu den Isocyanatgruppen von 4% erreicht; der Gasvolumenstrom beginnt aufgrund der Stöchiometrie und der Reaktionsenthalpie signifikant zu steigen, aber die querschnittsgemittelte Strömungsgeschwindigkeit überschreitet infolge des größer werdenden Strömungsquerschnitts an keinem stromab gelegenen Ort die querschnittsgemittelte Anfangsgeschwindigkeit am Ort des genannten Umsatzes der Amingruppen zu den Isocyanatgruppen von 4%, d.h. bei einer Länge des Rohrreaktors von 350 mm. Der Verlauf der querschnittsgemittelten Strömungsgeschwindigkeit gegenüber der Länge des Rohrreaktors ist in Fig. 1 dargestellt. Dort zeigt sich anschaulich, dass die querschnittsgemittelte Strömungsgeschwindigkeit ab einem Umsatz der Amingruppen zu den Isocyanatgruppen von 4%, d.h. nach 350 mm Länge ab Düsenmündung, stets niedriger ist als bei dem Umsatz der Amingruppen zu den Isocyanatgruppen von 4%, d.h. bei der Länge von 350 mm. Bis zum weitestgehend vollständigen Abschluss der Umsetzung des 2,4-Toluylendiamins und seiner Folgeprodukte mit dem Phosgen ist bei einer 2,4-Toluylendiisocyanatausbeute von > 99,5% eine minimale Reaktionsraumlänge ab der Düsenmündung von nur 4,2 m erforderlich.

### Zur Erläuterung:

Fig. 1 zeigt den Verlauf der querschnittsgemittelten Strömungsgeschwindigkeit für die Beispiele 2 und 3. Dabei ist die Position der Düsenmündung jeweils bei einer Länge des Rohrreaktors x = 0 m. Die querschnittsgemittelte Strömungsgeschwindigkeit v ist in m/s angegeben. B2 und B3 kennzeichnen Beispiel 2 und Beispiel 3.

## Patentansprüche

1. Verfahren zur Herstellung von aromatischen Isocyanaten durch Umsetzung von aromatischen primären Aminen mit Phosgen in der Gasphase, **dadurch gekennzeichnet, dass** Phosgen und die primären aromatischen Amine oberhalb der Siedetemperatur der Amine in einem Reaktor enthaltend einen zur Strömungsrichtung im Wesentlichen rotationssymmetrischen Reaktionsraum umgesetzt werden, wobei
a) die querschnittsgemittelte Strömungsgeschwindigkeit des Reaktionsgemisches längs der Achse des im Wesentlichen rotationssymmetrischen Reaktionsraumes in dem Abschnitt des Reaktionsraums, in dem der Umsatz der Amingruppen zu den Isocyanatgruppen zwischen 4 und 80 % liegt, maximal 8 m/s beträgt und
b) die querschnittsgemittelte Strömungsgeschwindigkeit des Reaktionsgemisches längs der Achse des im Wesentlichen rotationssymmetrischen Reaktionsraumes in dem Abschnitt des Reaktionsraums, in dem der Umsatz der Amingruppen zu den Isocyanatgruppen zwischen 4 und 80 % liegt, stets unterhalb der querschnittsgemittelten Strömungsgeschwindigkeit am Anfang dieses Abschnittes liegt.

2. Verfahren nach Anspruch 1, **dadurch gekennzeichnet, dass** der Reaktionsraum in dem Abschnitt des Reaktionsraums, in dem der Umsatz der Amingruppen zu den Isocyanatgruppen zwischen 4 und 80 % liegt, mindestens eine Erweiterung der durchströmten Querschnittsfläche des Reaktionsraums aufweist.

3. Verfahren nach Anspruch 1, **dadurch gekennzeichnet, dass** der Reaktionsraum in dem Abschnitt des Reaktionsraums, in dem der Umsatz der Amingruppen zu den Isocyanatgruppen zwischen 4 und 80 % liegt, mindestens eine konusförmige Erweiterung der durchströmten Querschnittsfläche des Reaktionsraums aufweist, wobei die konusförmige Erweiterung einen Aufweitungshalbwinkel von ≤ 6°, bevorzugt ≤ 3,5°, besonders bevorzugt ≤ 2°, aufweist.

4. Verfahren nach Anspruch 1, **dadurch gekennzeichnet, dass** der Reaktor im Anschluss an den Abschnitt des Reaktionsraums, in dem der Umsatz der Amingruppen zu den Isocyanatgruppen zwischen 4 und 80 % liegt, einen im Wesentlichen rotationssymmetrischen Reaktionsraum mit konstanter und / oder sich erweiternder durchströmter Querschnittsfläche aufweist.

5. Verfahren nach Anspruch 1, **dadurch gekennzeichnet, dass** die primärem aromatischen Amine und Phosgen vermischt und miteinander umgesetzt werden, wobei die Vermischung und die Umsetzung jeweils isotherm oder zumindest teilweise adiabatisch durchgeführt werden.

6. Verfahren nach Anspruch 1, **dadurch gekennzeichnet, dass** der im Wesentlichen rotationssymmetrische Reaktionsraum zumindest eine Zone aufweist, in der zum Abbruch der Umsetzung zumindest eine Flüssigkeit eingedüst wird.

7. Verfahren nach Anspruch 6, **dadurch gekennzeichnet, dass** die Querschnittsfläche der zumindest einen Zone, in der zum Abbruch der Umsetzung zumindest eine Flüssigkeit eingedüst wird, genauso groß oder größer ist als die Querschnittsfläche des Reaktionsraums, in dem die Umsetzung stattfindet.

8. Verfahren nach Anspruch 1, **dadurch gekennzeichnet, dass** das Phosgen und die Amine dem Reaktionsraum gasförmig und nach dem Strahlmischerprinzip zugeführt werden.

9. Verfahren nach Anspruch 1, **dadurch gekennzeichnet, dass** die in den Reaktionsraum eingespeisten Amin-enthaltenden bzw. Phosgen-enthaltenden Stoffströme dem Reaktionsraum mit einem Verhältnis der mittleren Strömungsgeschwindigkeiten von 2-12 zugeführt werden.

10. Verfahren nach Anspruch 8, **dadurch gekennzeichnet, dass** der oder die Stoffströme, welche die Amine enthalten, mit einer höheren mittleren Strömungsgeschwindigkeit in den Reaktionsraum eintreten als der oder die Stoffströme, welche das Phosgen enthalten.

11. Verfahren nach Anspruch 1, **dadurch gekennzeichnet, dass** als Amine Diamine eingesetzt werden.

12. Verfahren nach Anspruch 1, **dadurch gekennzeichnet, dass** als Diamine 2,4-Toluylendiamin, 2,6-Toluylendiamin oder Gemische daraus eingesetzt werden.

13. Verfahren nach Anspruch 1, **dadurch gekennzeichnet, dass** der Reaktor eine Durchsatzkapazität von > 1 t an Aminen / h aufweist.

## Claims

1. Process for preparing aromatic isocyanates by reaction of aromatic primary amines with phosgene in the gas phase, **characterized in that** phosgene and the primary aromatic amines are reacted at above the boiling point of the amines in a reactor containing a reaction space which is essentially rotationally symmetric about the flow direction, where
a) the flow velocity of the reaction mixture averaged over the cross section along the axis of the essentially rotationally symmetric reaction space is not more than 8 m/s in the section of the reaction space in which the conversion of the amine groups into isocyanate groups is in the range from 4 to 80% and
b) the flow velocity of the reaction mixture averaged over the cross section along the axis of the essentially rotationally symmetric reaction space in the section of the reaction space in which the conversion of the amine groups into isocyanate groups is in the range from 4 to 80% is always below the flow velocity averaged over the cross section at the beginning of this section.

2. Process according to Claim 1, **characterized in that** the reaction space has at least one widening of the cross-section area through which flow occurs of the reaction space in the section of the reaction space in which the conversion of the amine groups into isocyanate groups is in the range from 4 to 80%.

3. Process according to Claim 1, **characterized in that** the reaction space has at least one conical widening of the cross-sectional area through which flow occurs of the reaction space in the section of the reaction space in which the conversion of the amine groups into isocyanate groups is in the range from 4 to 80%, where the conical widening has a widening half angle of ≤ 6°, preferably ≤ 3.5°, particularly preferably ≤ 2°.

4. Process according to Claim 1, **characterized in that** the reactor has an essentially rotationally symmetric reaction space having a constant and/or widening cross-sectional area through which flow occurs after the section of the reaction space in which the conversion of the amine groups into isocyanate groups is in the range from 4 to 80%.

5. Process according to Claim 1, **characterized in that** the primary aromatic amines and phosgene are mixed and reacted with one another, where the mixing and the reaction are in each case carried out isothermally or at least partially adiabatically.

6. Process according to Claim 1, **characterized in that** the essentially rotationally symmetric reaction space has at least one zone in which at least one liquid is sprayed in to stop the reaction.

7. Process according to Claim 6, **characterized in that** the cross-sectional area of the at least one zone into which at least one liquid is sprayed to stop the reaction is equal to or greater than the cross-sectional area of the reaction space in which the reaction takes place.

8. Process according to Claim 1, **characterized in that** the phosgene and the amines are fed in gaseous form and according to the jet mixer principle into the reaction space.

9. Process according to Claim 1, **characterized in that** the amine-containing and phosgene-containing streams fed into the reaction space are introduced into the reaction space with a ratio of the average flow velocities of 2-12.

10. Process according to Claim 8, **characterized in that** the stream or streams containing the amines enter the reaction space at a higher average flow velocity than the stream or streams containing the phosgene.

11. Process according to Claim 1, **characterized in that** diamines are used as amines.

12. Process according to Claim 1, **characterized in that** 2,4-toluenediamine, 2,6-toluenediamine or mixtures thereof are used as diamines.

13. Process according to Claim 1, **characterized in that** the reactor has a throughput capacity of > 1 tonne of amines/h.

## Revendications

1. Procédé pour la production d'isocyanates aromatiques par mise en réaction d'amines primaires aromatiques avec du phosgène dans la phase gazeuse, **caractérisé en ce qu'**on fait réagir le phosgène et les amines aromatiques primaires au-dessus de la température d'ébullition des amines dans un réacteur comportant une chambre de réaction essentiellement à symétrie de révolution par rapport à la direction de l'écoulement,
a) la vitesse d'écoulement, établie en moyenne sur la section transversale, du mélange réactionnel le long de l'axe de la chambre de réaction essentiellement à symétrie de révolution, dans le segment de la chambre de réaction dans lequel le degré de conversion des groupes amino en les groupes isocyanate est compris entre 4 et 80 %, est au maximum de 8 m/s et
b) la vitesse d'écoulement, établie en moyenne sur la section transversale, du mélange réactionnel le long de l'axe de la chambre de réaction essentiellement à symétrie de révolution, dans le segment de la chambre de réaction dans lequel le degré de conversion des groupes amino en les groupes isocyanate est compris entre 4 et 80 %, est inférieure à la vitesse d'écoulement, établie en moyenne sur la section transversale, au début de ce segment.

2. Procédé selon la revendication 1, **caractérisé en ce que** dans le segment de la chambre de réaction dans lequel le degré de conversion des groupes amino en les groupes isocyanate est compris entre 4 et 80 %, la chambre de réaction présente au moins un élargissement de la surface de la section transversale, traversée par le courant, de la chambre de réaction.

3. Procédé selon la revendication 1, **caractérisé en ce que** dans le segment de la chambre de réaction dans lequel le degré de conversion des groupes amino en les groupes isocyanate est compris entre 4 et 80 %, la chambre de réaction présente au moins un élargissement conique de la surface de la section transversale, traversée par le courant, de la chambre de réaction, l'élargissement conique présentant un demi-angle d'élargissement de ≤ 6°, de préférence ≤ 3,5°, de façon particulièrement préférée ≤ 2°.

4. Procédé selon la revendication 1, **caractérisé en ce qu'**à la suite du segment de la chambre de réaction dans lequel le degré de conversion des groupes amino en les groupes isocyanate est compris entre 4 et 80%, le réacteur présente une chambre de réaction essentiellement à symétrie de révolution, à surface de section transversale, traversée par le courant, constante et/ou allant en s'élargissant.

5. Procédé selon la revendication 1, **caractérisé en ce que** les amines aromatiques primaires et le phosgène sont mélangés et mis en réaction entre eux, le mélange et la réaction étant effectués chacun en mode isothermique ou au moins partiellement adiabatique.

6. Procédé selon la revendication 1, **caractérisé en ce que** la chambre de réaction, essentiellement à symétrie de révolution, comporte au moins une zone dans laquelle est injecté au moins un liquide pour l'interruption de la réaction.

7. Procédé selon la revendication 6, **caractérisé en ce que** la surface de section transversale de ladite au moins une zone, dans laquelle est injecté au moins un liquide pour l'interruption de la réaction, est aussi grande ou plus grande que la surface de section transversale de la chambre de réaction, dans laquelle a lieu la réaction.

8. Procédé selon la revendication 1, **caractérisé en ce que** le phosgène et les amines sont introduits sous forme de gaz et selon le principe du mélangeur à jet.

9. Procédé selon la revendication 1, **caractérisé en ce que** les courants de substances contenant les amines ou, respectivement, contenant le phosgène, introduits dans la chambre de réaction, sont introduits en un rapport des vitesses moyennes d'écoulement de 2 - 12.

10. Procédé selon la revendication 8, **caractérisé en ce que** le ou les courants de substances qui contiennent les amines entrent dans la chambre de réaction à une plus grande vitesse moyenne d'écoulement que le ou les courants de substances qui contiennent le phosgène.

11. Procédé selon la revendication 1, **caractérisé en ce qu'**on utilise comme amines des diamines.

12. Procédé selon la revendication 1, **caractérisé en ce qu'**on utilise comme diamines la 2,4-toluylènediamine, la 2,6-toluylènediamine ou des mélanges de ces amines.

13. Procédé selon la revendication 1, **caractérisé en ce que** le réacteur présente une capacité de débit de > 1 t d'amines/h/.
